# EUROPEAN PATENT SPECIFICATION

(11) **EP 0 513 221 B1**
(45) Date of publication and mention of the grant of the patent: **13.08.1997**
(21) Application number: 91904634.2
(22) Date of filing: 05.02.1991
(51) Int. Cl.: A61H 7/00, A61N 1/32

(54) **DEVICE FOR LOOSENING SKIN TISSUE**
VORRICHTUNG ZUM LÖSEN VON HAUTGEWEBE
DISPOSITIF PERMETTANT DE DETENDRE LES TISSUS DE LA PEAU

(30) Priority: 11.02.1990 US 483405
(43) Date of publication of application: 19.11.1992
(73) Proprietor: SONO THERAPY INSTITUTE, INC., Phoenix, AZ 85016 (US)
(72) Inventor: PITZEN, Sylvester, A., Phoenix, AZ 85012 (US)
(74) Representative: Jones, Ian
(86) International application number: US9100772
(87) International publication number: WO9111980

(56) References cited:
- EP-A- 0 107 258
- DE-A- 2 815 643
- DE-A- 3 537 211
- FR-A- 1 480 327
- FR-A- 2 585 242
- US-A- 3 872 859
- US-A- 4 175 551

## Description

This invention primarily relates to a device for loosening connective skin tissue and stimulating blood circulation particularly beneficial in the rejuvenation of the human scalp as an aid to establishing or improving hair growth.

Hair loss resulting in male pattern baldness has come to be accepted as a fact of life in a large percentage of men and even some women. Attempts at finding a solution to male pattern baldness are almost endless. Even though many have claimed to have solved the problem, so far as known, there has yet to be found a solution that will truly reverse the process of hair loss resulting in male pattern baldness.

The present invention resulted from a practical analysis of the conditions that caused hair loss. In general, hair is generated out of hair roots and follicles that are rooted deep inside the several skin layers that overlie the skull bone. Arteries and veins within these multiple skin layers, which are also comprised of connective tissue and nerves, provide blood supply to the hair roots and follicles which is very necessary for the follicles to remain elongated and to maintain hair growth. When there is an increase in connective tissue cross-linkage and blood supply is restricted, the follicle shortens and hair growth stops. Each hair on a person's scalp has a short life cycle. A full head of hair is generally evidence that the follicles are actively producing hair. As blood supply follicle elongation and natural nourishment to the follicles begins to wane, so too does the hair growth process and male pattern baldness rapidly develops.

The analysis now shifts to the question as to what causes the reduction of blood supply to the hair root and follicles of a person's scalp, why does hair loss follow genetic patterns, why does hair loss assume different patterns on different persons' scalps, why is it prevalent in men over women, and why does it typically (but not always) begin developing in males in the early to mid-twenties.

The answers to all of these questions are believed provided by a consideration of the scalps of persons who develop male pattern baldness versus those who do not. From studies of numerous case histories and the results from extensive testing of the concept embodied in the present invention, it is theorized that male pattern baldness largely develops from a combination of three factors.

First is the vascular make-up of the individual. It is known that some individuals have better blood circulation than others. One person may practice horrendous eating and/or grooming habits and never have a blood circulation problem whereas those same habits will cause serious problems for other persons. An individual who naturally tends to have poor blood circulation may be a likely candidate for male pattern baldness.

Second is the physical structure of a person's scalp. A person having a "tight" scalp will tend to have skin that is more taut across the top of the head. This condition coupled with the aging process and stress causes non-elasticity in the scalp tissue resulting from increase cross-linkage of connective tissue. Different parts of the scalp will have different degrees of tightness depending on the shape of the skull. As will be explained herein after, skin that is stretched tightly over the skull will more likely experience non-elasticity and a general breakdown of the subcutaneous fat and restriction of the natural nutritional process including nerve stimulation and blood flow, all of which otherwise "feeds" the hair root and hair follicle.

The third factor is aging. Skin, like all other organs of the body, goes through an aging process. During the first 10-15 years, from birth to adolescence, a person's skin is growing and is very pliable. As maturity sets in, the skin starts to lose elasticity. In those persons where blood circulation in the scalp is more likely to be restricted genetically, where shape of the skull is more likely to induce a tightness of the skin, and where hair follicles are simply not as elongated or healthy; as aging sets in, the elasticity of the skin is reduced, connective tissue becomes dense, blood circulation to the hair root and follicle is further restricted, and hair growth is slowed or inhibited.

The present invention is an improvement of the device disclosed in U. S. Patent No. 3,872,859. The concepts explained in that patent are largely applicable to the present invention. The precept of that invention as well as the present invention is that hair root and hair follicles are not created by either the invention of that patent or the present invention. The aging process is not reversed nor is the bone structure or genetic make-up altered in any respect. To the extent that the hair roots and hair follicles are simply dormant as a result of increased density of connective tissue and inadequate blood supply which in turn may be the result of heredity, aging, shape of skull and skin tightening, hair growth can be rejuvenated by loosening with massage and electrical impulse stimulation, the skin layers that overlie the skull and in which the blood vessels subcutaneous fat and other nutritional processes along with the hair follicles reside.

Previously, limited success was experienced through the application of low voltage electrical stimulation of the skin layers of the scalp coupled with massage, as explained in the above-mentioned patent. However, this success was limited in that many treatments over a long period of time were required. In many cases, the treatment was only able to retard hair loss and in others provided little or no increase in hair growth rejuvenation. A significant number of cases did, however, experience rejuvenated hair growth which proved to the inventor that the adopted precept as explained above was valid. It is believed that the lack of success was a result of electrical impulse fluctuations and necessary refinements to the massage technique(s). The aging process is ongoing and in those subjects where deterioration of follicle elongation and/or blood circulation outpaced the rejuvenation process, failure was eminent.

Whereas the primary focus of the invention is directed to hair growth, the loosening of connective tissue and improved blood circulation is found to have benefits for other physical impairments as well. Injuries or disease affecting body tissue may similarly reduce elasticity of tissue and create blood circulation problems. As reported in the New Scientist Journal, May 17, 1979 issue:
"If a tensional stress is imposed on connective tissue over a long period, the fibroblasts which make up most of its bulk orient themselves along the lines of stress and begin to multiply more rapidly. They produce more collagen, the fibrous infrastructure of connective tissue. The extra fibers reduce the elasticity of the tissue. As collagen is fairly resistant to enzyme breakdown, these changes tend to be irreversible. The extra fibers take up space in the connective tissue of the muscle, and begin to encroach on the space normally occupied by nerves, blood and lymph vessels. As a result of this crowding, the tissue loses its elasticity and sometimes becomes painful when the muscle is set to work. The required work might then be attempted via another region of tissue, and the useful life of that region would be limited."

Applying the device discussed herein to such injured areas has been found to improve the healing process. However, benefits are not restricted to human subjects. Where a dog suffering from hip dysplasia was treated, the dog was observed to have freedom of movement not previously noted for many months prior to the treatment.

In general, the benefits of connective tissue loosening and improved blood circulation are endless. Even appearances are improved as one might expect from increasing blood circulation which in turn produces a more healthy appearing skin. Scalp and/or facial massage with the technique herein described is considered by some subjects to have provided wrinkle reduction and other cosmetic improvements. The technique, when applied to subjects suffering from various forms of arthritis, has reportedly resulted in improvement in range of motion and comfort in performing tasks which were otherwise painful. These and other yet unknown benefits are likely to result from connective tissue loosening and improved blood circulation generated by the present invention. Another less obvious application may be the treatment of problems that develop in space travel. Recent reports on the hazards of space travel suggest that inhibited red blood cell production and reduced muscle resistance may be a serious problem with persons spending long periods in a condition of weightlessness. The present invention or improvements or modifications thereto may provide the answer.

US 4175551 discloses a device for medical treatment of pain by electrical stimulation of the nervous system.

According to the present invention, there is provided an apparatus for massage therapy of a designated body portion of a subject by loosening connective tissue underlying the skin surface of said body portion, the apparatus comprising: a low voltage generator having first and second current conductors, the generator providing a variable voltage current at the first conductor relative to the second conductor; and first and second electrical contacts electrically coupled to the first and second conductors, respectively, the first contact including a smooth metallic portion electrically coupled to the first conductor and arranged for application to the designated body portion in massaging fashion, and the second contact being arranged for electrical coupling to a body portion remote from the designated body portion, characterised in that the variable voltage current oscillates in the range up to essentially 1.350 volts.

The present invention is an improvement over the apparatus disclosed in US Patent No. 3,872,859. Low voltage stimulation and simultaneous massage are believed to still be the key factors for achieving nerve stimulation and loosening the skin layers and connective tissue surrounding the blood vessels, hair roots and hair follicles and thereby enhancing blood flow to the follicles and hair root. However, through study and experimentation, the inventor came to appreciate that he was not fully realizing the benefit of the electrical stimulation.

As previously practiced, fluctuating current of 3-10 volts was generated across a pair of electrical contacts. One of the contacts was used as the massaging instrument and was encased in a padding of desired compressibility. The padding materials that were used were insulating materials and at least in part inhibited the flow of current. Yet a substantially stronger current could not feasibly be used.

As presently developed, a fluctuating electrical current of between -.250 and +1.350 volt is generated across a pair of electrical contacts. The contacts and padding have been modified so as to provide a compressible massaging pad that does not significantly inhibit the flow of current to the subject. Whereas padding materials are inherently current inhibiters, this problem was overcome by adopting highly conductive metal leads surrounded by cable shielding and encasing the contacts from the conductors in a highly absorbent padding material such as wool. The wool padding is moistened to render the wool padding conductive.

A further modification is in the modified treatment. In the previous method, one of the contacts was utilized as the massaging instrument with the other contact placed on the scalp in close proximity to the area being massaged. The electrical current between the contacts was largely passed across the surface skin as distinguished from the sub-layers of skin. As previously discussed, the follicles, blood supply and connective tissue are located in the sub-layers of skin and limited benefit was being derived from the electrical stimulation.

In the present method which uses the claimed device, the ground contact is preferably held in the subject's hand thereby directing passage of the current down through the sub-layers of skin.

The changes to the apparatus and method of U. S. Patent No. 3,872,859 have brought surprisingly improved results. Subjects who were experiencing slight rejuvenated hair growth now experience significant and more rapid hair growth. Those that experienced hair loss retardation now experience actual hair growth rejuvenation.

Although the theory has not been substantiated at present, it is also believed that administration of the treatment is enhanced by a perceived "operator feel". Operators utilizing the technique of the invention, experience a physical sense of increased resistance to the massaging action. This increased resistance is believed to occur in areas of taut skin and dense connective tissue. When not electrically stimulated, the entire scalp will seem to the operator to move at generally the same level of resistance. When stimulated by the electricity, there is a strong physical sensation that specific areas will not readily move under the massaging pad. It is theorized that the electrical impulse stimulation causes nerve-induced tightening of the connective tissue in those areas where the skin layers are already tight. The operator feels a more noticeable resistance to skin movement. These sensations were not being as frequently experienced under the prior method of the '859 patent and the resulting opportunity to focus treatment on the tight areas that are more readily sensed renders the treatment more effective. This sensation speeds up the effectiveness of the treatment which overcomes the otherwise ongoing deterioration of the scalp due to aging, heredity, and shape of skull.

The invention will be more fully understood by reference to the following detailed description and drawings.
Fig 1 is a schematic illustration of the apparatus of the invention being applied to a subject person;
Fig. 2 is an enlarged partial view in section of the scalp of the subject person of Fig. 1 illustrating the treatment being applied to that person;
Fig. 3 is a further enlarged schematic illustration of what is believed to take place within the skin layers of the scalp during the treatment of Figs. 1 and 2; and
Fig. 4 is a circuit diagram for the voltage producing device utilized in the apparatus of Fig. 1.

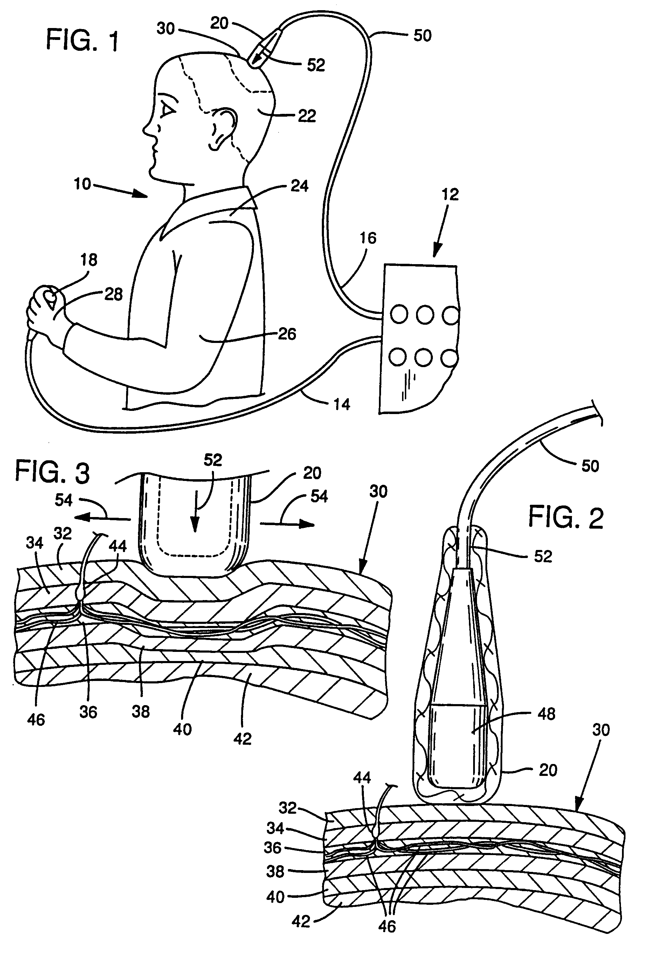

Referring to Fig. 1, a subject person 10 is being treated in accordance with the invention. A low voltage generating device 12 includes a pair of electrical current conductors 14, 16 surrounded by cable shielding and terminating in contact pads 18, 20. The power source for the generating device 12 is 110 AC household current which is transformed by the generating device 12 into pulsating or oscillating DC current.

It is believed that the voltage should be continuously varied to achieve maximum results and thus the circuitry in the generating device 12 is designed to generate numerous square wave voltage configurations, e.g. as many as 12 square waves each of a different frequency. These are turned on and off at various intervals (timed) with the lower frequencies timed slower than the higher frequencies.

Twelve frequencies of the machine are as follows:

| | | |
|---|---|---|
| Channel One | 4,000 HZ Timed | 48 HZ Per Minute |
| Channel Two | 5,000 HZ Timed | 52 HZ Per Minute |
| Channel Three | 6,000 HZ Timed | 68 HZ Per Minute |
| Channel Four | 7,000 HZ Timed | 75 HZ Per Minute |
| Channel Five | 8,500 HZ Timed | 90 HZ Per Minute |
| Channel Six | 9,500 HZ Timed | 120 HZ Per Minute |
| Channel Seven | 10,500 HZ Timed | 140 HZ Per Minute |
| Channel Eight | 12,500 HZ Timed | 160 HZ Per Minute |
| Channel Nine | 14,500 HZ Timed | 185 HZ Per Minute |
| Channel Ten | 15,500 HZ Timed | 200 HZ Per Minute |
| Channel Eleven | 17,500 HZ Timed | 215 HZ Per Minute |
| Channel Twelve | 18,500 HZ Timed | 225 HZ Per Minute |

The output of the device is variable from -.250 volt to approximately +1.350 volt.

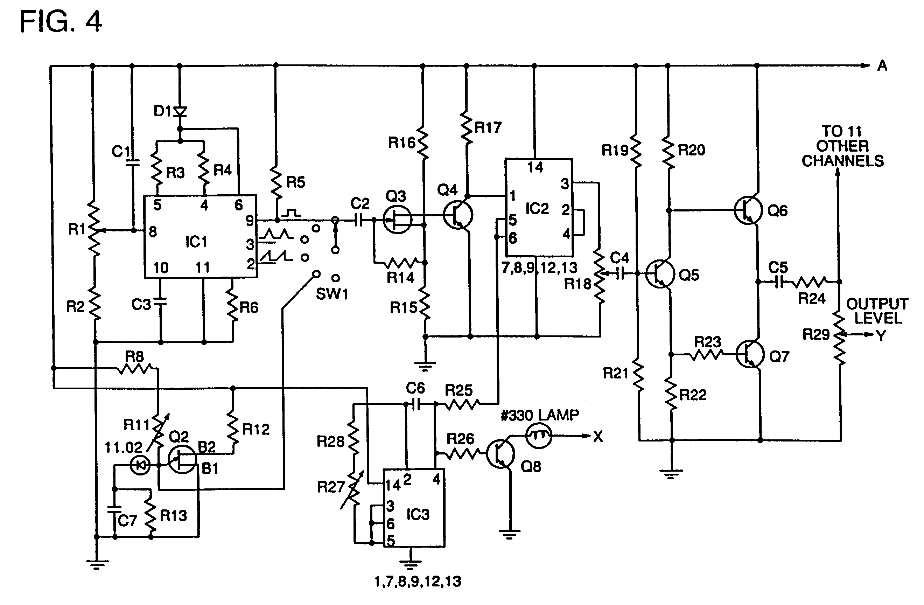

One form of circuitry was disclosed in the prior U. S. Patent No. 3,872,859. The preferred circuitry at present is illustrated in Fig. 4. A person skilled in electronics will have no problem in understanding the application of the circuitry of Fig. 4 and further description thereof is not deemed necessary and not explained in further detail.

Pad 18 is the ground terminal and completes the flow of electrical current being conveyed to the person's body from contact pad 20. The electrical current from pad 20 is passed into the subject person's scalp 30, down through the person's head 22, shoulder 24 and arm 26 to pad 18 which is being gripped by the person's hand 28.

Reference is now made to Figs. 2 and 3 which schematically illustrate the treatment of the subject person's scalp 30 with the apparatus of the invention.

The various skin layers and connective tissue that make up the scalp are what is referred to as the scarf skin layer 32, the true skin layer 34, the adipose tissue layer 36, the galea layer 38, and the loose connected tissue layer 40. These skin layers overlie the skull 42. Embedded in the skin layers at the bottom of the true skin layer 34 is the hair follicle 44. Just below the follicle 44 are the blood vessels and arteries indicated generally by reference 46.

As explained in Patent No. 3,872,859, the aging process causes a thickening and tightening of the skin layers, particularly the underlying galea layer 38 and loose connected tissue layer 40. This aging process tends to restrict blood flow and nerve action through the blood vessels and arteries which in turn causes a shrinking of the hair follicles. The shrinking or shortening of the follicles causes a withdrawal of the hair root and follicles from the blood vessels and arteries to further impede nourishment to the hair root and follicles. The gradual growth dormancy results in hair loss as previously explained.

Referring now to the treatment, it is the objective of the invention to cause a low voltage electrical variable impulse current to penetrate down through the skin layers and particularly down to the galea and connective tissue. Simultaneously, particularly where the operator is able to detect tightness of skin, the total skin thickness, i.e. all the skin layers, is "worked" to loosen the layers. Repeated massage and electrical impulse stimulation acts as nerve stimulation which facilitates a "reawakening" or reversal of the natural nutritional processes in the scalp which gradually cease to function properly, or on their own, over time-repeated treatment is utilized until the scalp regains its natural ability to "feed" the hair root and corresponding elongated hair follicle. The end result of this technique is that the scalp will be able to generate hair growth.

The present invention is a refinement of the apparatus of U. S. Patent No. 3,872,859 and it is believed pertinent for the reader to understand the deficiencies of this prior method and apparatus in order to gain a full appreciation of the present invention.

The prior massaging process was not sufficiently localized. For the present invention, it is important that pressure be applied to a small area of the scalp which simulates a clamping of the skin to the skull. The contact pad is specifically designed to apply this localized pressure. In addition to the electrical stimulation, the pad is worked from side to side but without moving the pad relative to the contacted surface of the skin. In response to the electrical stimulation and the working of the control pad, the underlying layers relax and move relative to the skull. Whereas it is believed the tightness is very much localized, the present refined method is believed to be far more effective than was the prior method of massage stimulation.

A second deficiency of the prior method is in the manner of electrically stimulating the scalp. The pair of contact pads were both placed on the scalp. The electrical current passing from one to the other was readily accomplished through the skin near the surface thereof. Whereas the tightness occurs in the deeper underlayers, the electrical stimulation was only partially effective. With the present invention, the ground contact is placed lower on the body so that the flow of electrical current is directed downward into and through the under layers of the skin. For convenience, the ground contact pad 18 is simply grasped by the subject person's hand 28 as illustrated in Fig. 1.

It is further believed that the prior apparatus was deficient in the way the contact pads were constructed. As explained in the prior patent, the electrical contact at the end of the electrical conductor was wrapped in conductive mesh, then in cotton batting and finally in cloth strips. The cotton batting and cloth strips were moistened so that the current from the mesh was conducted to the subject person's scalp. Two problems are believed to exist with this construction. First the mesh itself does not adequately conduct the electrical current from the end of the conductor to the cotton and cloth. Second the cotton does not retain moisture particularly well and rapidly dries out. What moisture is retained by the cotton is not spread evenly, i.e. the moisture settles in specified areas of the cotton and does not provide a reliable point-to-point conduction.

In the present invention, a highly conductive metal bulb 48 is connected to the conductor 52 within cable shielding 50. The bulb 48 has far greater surface area than the former wire conductor and transmits the current to the surrounding cloth material far better than the prior mesh. The bulb of the contact 20 is primarily copper coated with gold metal, or silver metal and then gold metal. The cloth material wrapped around the bulb 48 is wool. Wool has far better moisture retention and remains reliably conductive far longer than the cotton material of the prior apparatus. Bulb 48 of the ground contact 18 is preferably copper coated with silver metal, or coated with gold and then the silver metal. The outer coating of silver is preferred because experience has shown that gold is rubbed off due to handling by the subject person, whereas silver is still a good conductor and greater resistance to wear.

With the above refinement and the modification of the circuitry of apparatus 12 illustrated in Fig. 4, the method and apparatus as otherwise explained in U. S. Patent No. 3,872,859 is generally applicable. The twelve square wave circuits generate a continually varying voltage of between -.250 volt and +1.350 volt. The operator grasps the contact 20 and applies pressure against the scalp in the manner of Figs. 1-3 as indicated by arrow 52, while the subject person holds the ground contact 18 as shown in Fig. 1. As indicated by arrow 54 in Fig. 3, the contact 20 is worked from side to side while maintaining pressure 52. This action is believed to far exceed the benefits of the method described in the prior patent. Furthermore, although applicant does not wish to be bound to this theory, there is a perceived feel of resistance to the working action at specific locations on the scalp which appears to be generated by the undesirable tight skin areas. This perception is prevalent with the voltage flowing to the skin as compared to when the current is turned off. It is believed that the skin reacts to the voltage causing it to tense or tighten. Where the underlayers have already tightened against the scalp, this tightening of the skin is magnified to the feel of tightness perceived by the operator. Thus, the electrical current is believed to provide a double benefit. First because the excitation coupled with "working" of the skin loosens the scalp, and second because the tight areas are more readily identified by the operator and the treatment can be focused on the areas of tight skin.

The invention is not limited to the specific apparatus disclosed but instead encompasses the broader definition of the claims appended hereto.

## Claims

1. An apparatus for massage therapy of a designated body portion of a subject by loosening connective tissue underlying the skin surface of said body portion, the apparatus comprising:
a low voltage generator (12) having first and second current conductors, the generator providing a variable voltage current at the first conductor relative to the second conductor; and
first and second electrical contacts (20,18) electrically coupled to the first and second conductors, respectively, the first contact (20) including a smooth metallic portion electrically coupled to the first conductor and arranged for application to the designated body portion in massaging fashion, and the second contact(18) being arranged for electrical coupling to a body portion remote from the designated body portion, characterised in that the variable voltage current oscillates in the range up to essentially 1.350 volts.

2. An apparatus as claimed in claim 1 wherein the variable voltage current comprises a plurality of periodic wave signals of different frequencies.

3. An apparatus as claimed in claim 2 wherein the different frequencies are in the range of 4,000 Hz to 18,500 Hz.

4. An apparatus as claimed in claim 2 or 3 wherein the different frequencies are twelve in number.

5. An apparatus as claimed in claim 2, 3 or 4 wherein the plurality of periodic wave signals are turned on and off at different intervals.

6. An apparatus as claimed in claim 5 wherein a lower frequency periodic wave signal is turned on and off at longer intervals than a higher frequency periodic wave signal.

7. An apparatus as claimed in claim 5 or 6 wherein the different predetermined intervals are in the range of 48 Hz per minute to 225 Hz per minute.

8. An apparatus as claimed in any one of claims 1 to 7 wherein the variable voltage current fluctuates between -0.25 and +1.35 volts.

9. An apparatus as claimed in any one of claims 2 to 8 wherein the periodic wave signals are square wave signals.

10. An apparatus as claimed in any one of claims 1 to 9 wherein the first and second contacts (20,18) each comprise a conductive metal bulb (48).

11. An apparatus as claimed in claim 10 wherein each metal bulb (48) is of copper.

12. An apparatus as claimed in claim 10 or 11 wherein each metal bulb (48) has a coating of gold or silver.

13. An apparatus as claimed in claim 12 wherein the metal bulb (48) of the first contact (20) has an outer coating of gold.

14. An apparatus as claimed in claim 12 or 13 wherein the second contact (18) has an outer coating of silver.

15. An apparatus as claimed in any one of claims 10 to 14 wherein the metal bulb (48) of the first electrode (20) is wrapped in woollen cloth.

16. An apparatus as claimed in any preceding claim wherein the second electrode (18) is shaped to be conveniently grasped by the human hand.

## Patentansprüche

1. Vorrichtung zur Massagetherapie eines bestimmten Körperteils eines Patienten durch Lösen von unter der Hautoberfläche des genannten Körperteils liegendem Bindegewebe, wobei die Vorrichtung folgendes umfaßt:
einen Niederspannungsgenerator (12) mit einem ersten und einem zweiten Stromleiter, wobei der Generator einen Strom mit variabler Spannung zu dem ersten Leiter relativ zu dem zweiten Leiter speist; und
einen ersten und einen zweiten elektrischen Kontakt (20, 18), die elektrisch jeweils mit dem ersten und dem zweiten Leiter gekoppelt sind, wobei der erste Kontakt (20) einen glatten Metallabschnitt aufweist, der elektrisch mit dem ersten Leiter gekoppelt ist und dazu dient, zur Massage an den bestimmten Körperteil angelegt zu werden, und wobei der zweite Kontakt (18) dazu dient, elektrisch an einen Körperteil entfernt von dem bestimmten Körperteil angeschlossen zu werden, dadurch gekennzeichnet, daß der Strom mit variabler Spannung im Bereich bis im wesentlichen 1,350 Volt schwankt.

2. Vorrichtung nach Anspruch 1, bei der der Strom mit variabler Spannung eine Mehrzahl von periodischen Wellensignalen unterschiedlicher Frequenzen umfaßt.

3. Vorrichtung nach Anspruch 2, bei der die unterschiedlichen Frequenzen im Bereich zwischen 4.000 und 18.500 Hz liegen.

4. Vorrichtung nach Anspruch 2 oder 3, wobei die Zahl der unterschiedlichen Frequenzen zwölf beträgt.

5. Vorrichtung nach Anspruch 2, 3 oder 4, bei der die Mehrzahl von periodischen Wellensignalen in unterschiedlichen Intervallen ein- und ausgeschaltet werden.

6. Vorrichtung nach Anspruch 5, bei der ein periodisches Wellensignal einer niedrigeren Frequenz in längeren Intervallen ein- und ausgeschaltet wird als ein periodisches Wellensignal einer höheren Frequenz.

7. Vorrichtung nach Anspruch 5 oder 6, bei der die unterschiedlichen vorbestimmten Intervalle im Bereich zwischen 48 Hz pro Minute und 225 Hz pro Minute liegen.

8. Vorrichtung nach einem der Ansprüche 1 bis 7, bei der die variable Spannung zwischen -0,25 und +1,35 Volt schwankt.

9. Vorrichtung nach einem der Ansprüche 2 bis 8, bei der die periodischen Wellensignale Rechteckwellensignale sind.

10. Vorrichtung nach einem der Ansprüche 1 bis 9, bei der der erste und der zweite Kontakt (20, 18) jeweils eine leitende Metallbirne (48) umfaßt.

11. Vorrichtung nach Anspruch 10, bei der jede Metallbirne (48) aus Kupfer ist.

12. Vorrichtung nach Anspruch 10 oder 11, bei der jede Metallbirne (48) eine Beschichtung aus Gold oder Silber hat.

13. Vorrichtung nach Anspruch 12, bei der die Metallbirne (48) des ersten Kontaktes (20) eine Außenschicht aus Gold hat.

14. Vorrichtung nach Anspruch 12 oder 13, bei der der zweite Kontakt (18) eine Außenschicht aus Silber hat.

15. Vorrichtung nach einem der Ansprüche 10 bis 14, bei der die Metallbirne (48) der ersten Elektrode (20) in ein Wolltuch gehüllt ist.

16. Vorrichtung nach einem der vorherigen Ansprüche, bei der die zweite Elektrode (18) so geformt ist, daß sie von der menschlichen Hand leicht ergriffen werden kann.

## Revendications

1. Appareil de soins par massage d'une partie du corps déterminée d'un sujet en relâchant le tissu conjonctif sous-jacent à la surface de la peau de ladite partie du corps, l'appareil comprenant :
un générateur basse tension (12) ayant des premier et deuxième conducteurs de courant, le générateur délivrant un courant à tension variable au niveau du premier conducteur par rapport au deuxième conducteur ; et
des premier et deuxième contacts électriques (20, 18) couplés électriquement aux premier et deuxième conducteurs, respectivement, le premier contact (20) comprenant une partie métallique lisse couplée électriquement au premier conducteur et agencée pour être appliquée à la partie du corps déterminée en réalisant un massage, et le deuxième contact (18) étant agencé en vue du couplage électrique à une partie du corps éloignée de la partie du corps déterminée, caractérisé en ce que le courant à tension variable oscille essentiellement dans une plage de l'ordre de 1,350 volt.

2. Appareil selon la revendication 1, dans lequel le courant à tension variable comprend une pluralité de signaux d'onde périodique de fréquences différentes.

3. Appareil selon la revendication 2, dans lequel les différentes fréquences sont dans la plage de 4000 Hz à 18500 Hz.

4. Appareil selon la revendication 2 ou 3, dans lequel les différentes fréquences sont au nombre de douze.

5. Appareil selon la revendication 2, 3 ou 4, dans lequel la pluralité de signaux d'onde périodique sont envoyés et interrompus à différents intervalles.

6. Appareil selon la revendication 5, dans lequel un signal d'onde périodique de fréquence inférieure est envoyé et interrompu à des intervalles plus longs qu'un signal d'onde périodique de fréquence supérieure.

7. Appareil selon la revendication 5 ou 6, dans lequel les différents intervalles prédéterminés sont dans la plage de 48 Hz par minute à 225 Hz par minute.

8. Appareil selon l'une quelconque des revendications 1 à 7, dans lequel le courant de tension variable fluctue entre -0,25 et +1,35 volt.

9. Appareil selon l'une quelconque des revendications 2 à 8, dans lequel les signaux d'onde périodique sont des signaux d'onde carrée.

10. Appareil selon l'une quelconque des revendications 1 à 9, dans lequel les premier et deuxième contacts (20, 18) comprennent chacun un bulbe métallique conducteur (48).

11. Appareil selon la revendication 10, dans lequel chaque bulbe métallique (48) est en cuivre.

12. Appareil selon la revendication 10 ou 11, dans lequel chaque bulbe métallique (48) a un revêtement d'or ou d'argent.

13. Appareil selon la revendication 12, dans lequel le bulbe métallique (48) du premier contact (20) a un revêtement extérieur d'or.

14. Appareil selon la revendication 12 ou 13, dans lequel le deuxième contact (18) a un revêtement extérieur d'argent.

15. Appareil selon l'une quelconque des revendications 10 à 14, dans lequel le bulbe métallique (48) de la première électrode (20) est entouré dans un tissu de laine.

16. Appareil selon l'une quelconque des revendications précédentes, dans lequel la deuxième électrode (18) est profilée de manière à offrir une prise commode pour la main d'une personne.
